# EUROPEAN PATENT SPECIFICATION

(11) **EP 1 967 079 B1**
(45) Date of publication and mention of the grant of the patent: **27.10.2010**
(21) Application number: 08004242.7
(22) Date of filing: 07.03.2008
(51) Int. Cl.: A23L 1/212, A61K 8/97, A61K 36/185, A23K 1/14, A23K 1/16

(54) **Process for preparing pomegranate extracts**
Verfahren zur Herstellung von Granatapfelextrakten
Processus pour préparer des extraits de grenade

(30) Priority: 08.03.2007 EP 07004765
(43) Date of publication of application: 10.09.2008
(73) Proprietor: Probelte Pharma, S.A., 30100 Espinardo Murcia (ES)
(72) Inventor: López Más, José A., 30840 Alhama de Murcia, Murcia (ES); Streitenberger, Sergio A., 30120 El Palmar, Murcia (ES); Peñalver Mellado, Marcos, 30007 Murcia, Murcia (ES); Martínez Ortis, Pedro, 30150 La Alberca, Murcia (ES)
(74) Representative: Gislon, Gabriele

(56) References cited:
- EP-A- 0 406 760
- WO-A-2004/056444
- WO-A-2005/096840
- WO-A-2005/097106
- KR-A- 20020 097 070
- US-A- 4 962 094
- US-A1- 2003 165 456
- US-A1- 2006 025 353
- US-A1- 2007 031 332
- MAYER W ET AL: "PUNICALAGIN UND PUNICALIN, ZWEI GERBSTOFFE AUS DEN SCHALEN DER GRANATAEPFEL//PUNICALAGIN AND PUNICALIN, TWO TANNINS FROM POMEGRANATE PEEL" JUSTUS LIEBIGS ANNALEN DER CHEMIE, VERLAG CHEMIE GMBH. WEINHEIM, no. 11/12, 1 January 1977 (1977-01-01), pages 1976-1986, XP009015975 ISSN: 0075-4617
- CERDA BEGONA ET AL: "Repeated oral administration of high doses of the pomegranate ellagitannin punicalagin to rats for 37 days is not toxic." JOURNAL OF AGRICULTURAL AND FOOD CHEMISTRY, vol. 51, no. 11, 21 May 2003 (2003-05-21), pages 3493-3501, XP002448624 ISSN: 0021-8561

## Description

The present invention relates to a process for preparing pomegranate extracts and to said extracts. More particularly, the invention relates to the production of ellagitannins-containing products to be used as a source of pomegranate ellagitannins in food, medical and cosmetic industries. Tannins are plant polyphenols including two categories of compounds, condensed tannins ,proanthocyanidins, and hydrolizable tannins, including gallotannins (esters of gallic acid linked to a sugar core) and ellagitannins (ester of ellagic acid also linked to a sugar core). Therefore, ellagitannins are hydrolizable tannins including punicalagins and other derivatives of ellagic acid such as ellagic acid glucoside and ellagic acid rhamnoside.

Among pomegranate ellagitannins, punicalagins are the major fruit husk tannin. Punicalagins α and β anomers are mainly present in pomegranate husk, and they pass to the primary juice during pressing for juice extraction. Punicalagins α and β have the following formula:

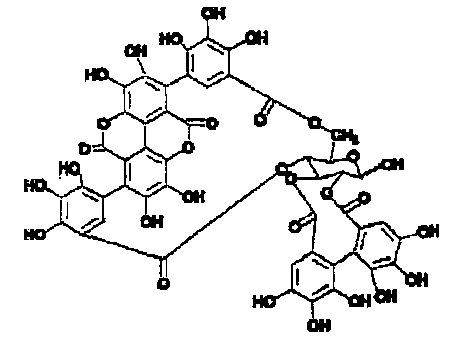

Recent studies showed that pomegranate juice and pomegranate extracts, contain a number of bioactive compounds, particularly polyphenols; among these polyphenols, ellagitannins are of outstanding biological significance in view of their antioxidant, antimicrobial and radical scavenging activity.

Studies have shown that pomegranate juice has the higher antioxidant properties than any other drink, such as blueberry juice, cranberry juice, green tea or red wine. Punicalagins α and β anomers are predominant ellagitannins in pomegranate juice, and the major responsible of antioxidant properties of the juice. Nevertheless, research pointed out that combination of punicalagins α and β with other phytochemicals such as ellagic acid and its glycosidated derivatives and other minor pomegranate ellagitannins produces an important synergistic effect enhancing healthy benefits of the juice.

Production of extracts containing a wide range of ellagitannins and phytochemicals from pomegranate was and is actively investigated; an efficient extraction process could be very profitable.

A recent profusion of pomegranate extracts, standardized to 40 % ellagic acid, has appeared in the market. However, the amount of ellagic acid per se is not relevant, because it was found that when free ellagic acid is administrated orally, it is poorly absorbed. In fact, numerous studies concluded that synergy among the various pomegranate phytochemicals and not simply the concentration of ellagic acid, is a key factor for assessing the healthy benefits of pomegranate extracts.

US 2006/0211635 discloses purification of pomegranate ellagitannins from fruit husk, a by-product of the commercial juice industry. The process includes the steps of preparing, by e.g. blending an aqueous suspension from pomegranate husk, removing the suspended solids from the aqueous solution, adsorbing the ellagitannins onto a resin surface (e.g. XAD-16 resin) from the aqueous solution, eluting the resin surface with ethanol or methanol, and evaporating the solvent.

Similarly WO2005/097106 and the corresponding paper "Rapid large scale purification of ellagitannins from pomegranate husk, a by product of the commercial juice industry" Separation and Purification Technology, ELSEVIER SCIENCE 41, n. 1, Jan. 2005, pages 49-55_describe the extraction of punicalagins from the husk only and purification on an adsorption resin vacuum-aspirated column. The tannins adsorbed on the column are recovered by elution with MeOH.

These processes have the drawback of requiring the use of organic solvents, even toxic ones such as methanol, and of using only husks to avoid having lots of sugars in the extracted solution. Thus, this process generates an extract than lacks several important phytochemicals mainly present in the pomegranate juice fraction and contains traces of the organic solvents used.

WO2006/127832 discloses a method to produce a pomegranate extract from by-products of the juice industry. Solids from pomegranate, pericarp, inner membrane and seeds are mixed with an aqueous solution and heated optimally to 43-71 °C adding enzymes that partially degrade the pomegranate solids. After that, the process continues as follows: remaining insoluble solids are removed, by a variety of methods known in the art (e.g. microfiltration) to produce the extract containing pomegranate phytochemicals, and the resulting liquid extract is concentrated in an evaporator under vacuum, pasteurized, re-concentrated in the evaporator, cooled, blended and standardized. The above described process includes several thermical treatments where the extract is subjected to heating (2 h at 43-71 °C and pasteurization). Pasteurization of pomegranate juice produces free ellagic acid trough degradation of larger ellagitannins, such as punicalagins, diminishing the health benefits of drinking the juice. These steps generate several by-products that are difficult to remove and, in addition, the use of pomegranate solids coming from the juice industry generates an extract than doesn't contain some phytochemicals mainly present in the pomegranate juice fraction. For this reason WO2006/127832 discloses at one of the embodiments the combination of pomegranate extract and juice, that in fact provides a broad spectrum of the phytochemicals present in the whole pomegranate, but has the disadvantage of the high caloric value due to the sugars present in both the juice and the extract.

BALA et al. "Antimutagenic effect of Pomeganate (Punica granatum var. anardana) fruit extracts on direct acting and S9-dependent mutagens in Salmonella typhimurium" in J. of Plant Science Research, 8, n.1/4, 1992, pages 14-16, discusses antimutagenic effect of an aqueous and chloroform extract of pomegranate obtained by soaking crushed fruit in chloroform and water. Eventually, chloroform was evaporated and replaced by an equal volume of dimethysulphoxide.

US2007/031332_discloses the use of several different fruit extracts or plant extracts as a source of gallic acid for the inhibition of angiogenesis. Example 23 of the description discloses a process of pomegranate fruit extraction wherein the fruits are manually cut and crushed, extracted in deionized water at 1/15 w/v ratio with boiling for 30 min, separation of the liquid extract, concentration and freeze-drying of the crude extract, reconstitution of the crude extract with deionized water in 1/15 w/v ratio and purification on adsorption resin with elution with ethanol.

Cerdà Begoña "Evaluation of the bioavailability and metabolism in the rat of punicalagin, an antioxidant polyphenol from pomegranate juice" Eur. J. of Nutrition Jan 2003, 42, 1, pages 18-28 and in "Repeated oral administration of high doses of the pomegranate ellagitannin punicalgin to rats for 37 days is not toxic" J. of Agric. and Food, 51, 11, May 2003, pages 3493-3501 describes the extraction of punicalagin from a blend of water and pomegranate husk for 2 hours at room temperature, filtration through a solid-phase extraction, reverse chromatography on C-18 columns and elution with alcohol (MeOH),
and by semi-preparative HPLC with MeOH-acetic acid-water (page 3495 left col.). This product is substantially pure punicalagin,

Mayer W et al. in "Punicalagin und punicalin zwei gerbstoffe aus den schalen del granatäpfel", Justus Liebigs Annalen der Chemie, Verlag Chemie GmbH, Weinheim, 11/12, 1 January 1977, pages 1976-1986, discloses isolation from a mixture of tannins obtained from the peels of pomegranates, of the two crystalline tannins, named punicalagin and punicalin, by an extended series of chromatographic separations. It should be noticed that the isolation has only an analytical purpose, that is the physical-chemical characterization of punicalagin and punicalin obtained at laboratory scale.

Summarizing, the above mentioned techniques have the disadvantage that they are either using only husk and/or by-products of the juice industry, or applying harsh conditions that result in an extract rich in ellagic acid that requires methanol or ethanol for purification.

A further problem is that the known extraction processes do not always give the same results in the composition of the final extract; namely, the inventors found that, though following the same procedures, the quantity of punicalagins and ellagic acid in the final extract can vary dramatically.

There is therefore the need for a process of extraction of pomegranates that can give a final product that is rich in punicalagins and has a reduced amount of ellagic acid, and that can give constant and repeatable compositions of the final extract.

It is an aim of the present invention to solve the above mentioned problems and to provide a process of producing pomegranate extract that is simple, effective, not expensive, that can provide water soluble punicalagins rather than solvent soluble ellagic acid, and that can provide in a repeatable way a final (liquid or solid) product having the broad spectrum of the different natural organic chemicals which are present in the whole pomegranate fruit, e.g. both ellagitannins and anthocyanins. In other words, it is an aim of the present invention to obtain an extract that can be used to transfer the useful properties of the pomegranate to any preparation or food.

Such an aim is achieved by means of the present invention that provides a process according to claim 1.

According to the invention the process provides for an extraction in the absence of organic solvents, of the entire, i.e. the whole, pomegranate fruits; preferably the entire fruits, previously washed, are blended with a mixer, known in the art at room temperature. In the blending step the mixer blades chop up the fruit pieces to obtain a suspension, i.e a dispersion of fruit particles in water. The ratio water/pomegranantes is within the range 1:1 to 1:2 (liters/kg) and the blending/chopping is carried out until the fruits are homogeneously dispersed in water.

In a preferred aspect of the invention the fruits are treated, with an aqueous solution of an acid, at a pH within the range of 1.0 to 2.0. The inventors found that this treatment results in much more constant results in the final composition: namely, by treating with mineral acid the amount of ellagic acid is always kept below the value of 5% (w/w) of the final extract. It is believed that the acid treatment is an efficient inactivation step for the tannase enzyme and takes into account the ubiquitous presence of filamentous fungi in pomegranate, able to produce tannase both extracelullar and intracellular.

The acid treatment can be carried out before, during or after the extraction step, but before the purification step on columns. Preferably, the acid treatment is carried out during the extraction step, i.e. when the whole fruits are blended in water to provide an homogeneous fine dispersion.

According to this aspect of the invention, acid treatment water used for blending is previously acidified by adding an acid, preferably sulphuric acid, being the final acid concentration within the range 0 to 1 % (w/V water). The pH of the homogenised mixture that results from the blending is within the range of 1.0 to 2.0.

The pomegranate crude extract, if needed, is readjusted to pH within the range 3.5 to 5.0 by adding a base, preferably NaOH, and clarified by physical methods known in the art, e.g. by filtering and/or centrifuging, to remove the insoluble solids from the aqueous solution, and to obtain a clarified solution substantially free of insoluble solids.

According to the invention, the above extraction steps are followed by a purification step wherein the water solution, i.e. the product previously obtained, is loaded in a chromatographic column of a resin selected from adsorbent non-ionic resins to adsorb the compounds of interest that are subsequently recovered by elution from the adsorption column with an aqueous weakly basic solution, preferably a buffer solution, most preferably obtained with sodium bicarbonate buffer. The elution is completed by a step of displacing with demineralised water.

According to another preferred embodiment of the invention, the pomegranate crude extract is subjected to a thermal treatment, that can be carried out before, or after the clarification step, but in all the cases before the purification step on columns. This process entails the heat treatment of the crude product at a temperature not exceeding 100°C, preferably within the range from 70°C to 100°C and most preferably, in the range of 80°C to 90°C at a residence time within the range of 1 second to 120 second and most preferably in the range from 5 seconds to 30 seconds. The pH of the clarified aqueous solution that undergoes the heat treatment is within the range of 1.0 to 5.0 and most preferably in the range from 3.5 to 5.0.

According to a further aspect of the invention, the column purified liquid product obtained without or with thermal treatment during their processing, is concentrated by nanofiltration and/or reverse osmosis concentration, to remove water and the salts present in the solution.

According to a further step, after chromatographic purification and nanofiltration and/or reverse osmosis concentration, the resulting liquid product is brought to a solid form, e.g. by freeze-drying, vacuum rotaevaporation or spray drying, with or without carriers such as maltodextrines or soluble fiber.

A further object of the invention are the ellagitannins containing extracts as obtainable according to the above mentioned process. These products may be in solid form and are characterized by having a punicalagins content of at least 30% (w/w) a purity of at least 70%, a content in ellagic acid of less than 15 % (w/w) and a content in anthocyanins of at least 0.1 % (w/w) preferably of at least 1 %.

The invention provides several advantages over the prior art techniques.

First of all, the extraction step is carried out using whole (entire) pomegranate fruits and water, operating at room temperature, the resulting extraction of water-soluble ellagitannins is almost completed in about one hour, without significant formation of by-products such as free ellagic acid.

The acidic treatment results in the inactivation of tannase enzyme, preventing hydrolysis of the ellagitannins to ellagic acid. The extraction step, i.e. mechanical blending, in acidic water (water pH within the range of 0.5 to 1.5) allows to obtain, in a short time, a very good yield in water-soluble hydrolysable ellagitannins, by the fast inactivation of tannase. In fact filamentous fungi producing such enzyme are more commonly found attacking pomegranate fruits especially during their postharvest storage and further processing but infection by fungi may occur at any time during the growing season, at harvest time, during handling, storage, transport and marketing.

The extraction step, carried out according to the invention, provides for another advantage. The exclusive use of (acidic) water avoids the extraction of huge amounts of the slightly water-soluble ellagic acid. In fact, the extraction step results in a composition of the pomegranate extract that is completely soluble in water.

In addition, the purification step, makes use of a water solution, weakly basic, free from organic solvents, and of demineralized water, to recover the polyphenols adsorbed on the resin. This means that no organic solvents are contacted with the aqueous extract, thus obtaining a pomegranate extract that is particularly suitable to be used in the alimentary, cosmetic and pharmaceutical field. Elution with water also results in recovering the other polyphenols extracted from the pulp of the fruits and adsorbed onto the resin, so as to obtain a broad spectrum of the original beneficial compounds of the whole fruit.

Another advantage of the purification step, is that it allows to separate the sugar fraction from the ellagitannins and polyphenols fraction. This purification step provides a "sugar-free" pomegranate extract, with all of the benefits of the pomegranate but without the calories, characterized by a high content on punicalagins and very little ellagic acid, which can be directly concentrated and dried without the drawbacks associated with high sugar content extracts (e.g. sticky materials). Always according to the present invention, the possibility to obtain a solid final product that is not mixed with any carrier, for example maltodextrines, gives the opportunity to formulate pomegranate extract according to its final intended use and according to any formal requirement, possibly required.

Additionally, when pomegranate crude extract is subjected to the above disclosed thermal treatment inactivation of enzymes and/or microorganisms i.e. their spores is obtained. In such a way the pomegranate crude extract could be further processed without spoilage by microorganisms able to excrete neoformed tannase and other undesirable substances. Final pomegranate extract is completely free of tannase forming microorganisms and/or free extracelular enzyme able to hydrolyze complex ellagitannins, such as punicalagins, when the extract is incorporated into a food or beverage.

The invention will now be further disclosed in greater detail with reference to the enclosed non-limiting drawings wherein:
- Fig.1 is a schematic view of an apparatus not according to the invention;
- Fig. 2A is a HPLC chromatogram of a pomegranate extract obtained according to one embodiment of the invention process;
- Fig. 2B is a HPLC chromatogram of pure punicalagins anomers isolated from the pomegranate extract;
- Fig 2C is a HPLC chromatogram of commercially available ellagic acid;
- Fig. 3 is a comparison of 3 different HPLC chromatograms, showing the peak profiles characteristic of an extraction of pomegranate fruits attacked by filamentous fungi at different pHs (by acidification of the water used for the extraction);
- Fig. 4A and Fig. 4B are comparisons of the HPLC chromatograms of the results of thermal treatment in the process of the invention; and
- Fig. 5 is the HPLC chromatogram of the punicalin product obtained by enzymatic hydrolysis from the punicalagin product of the invention.

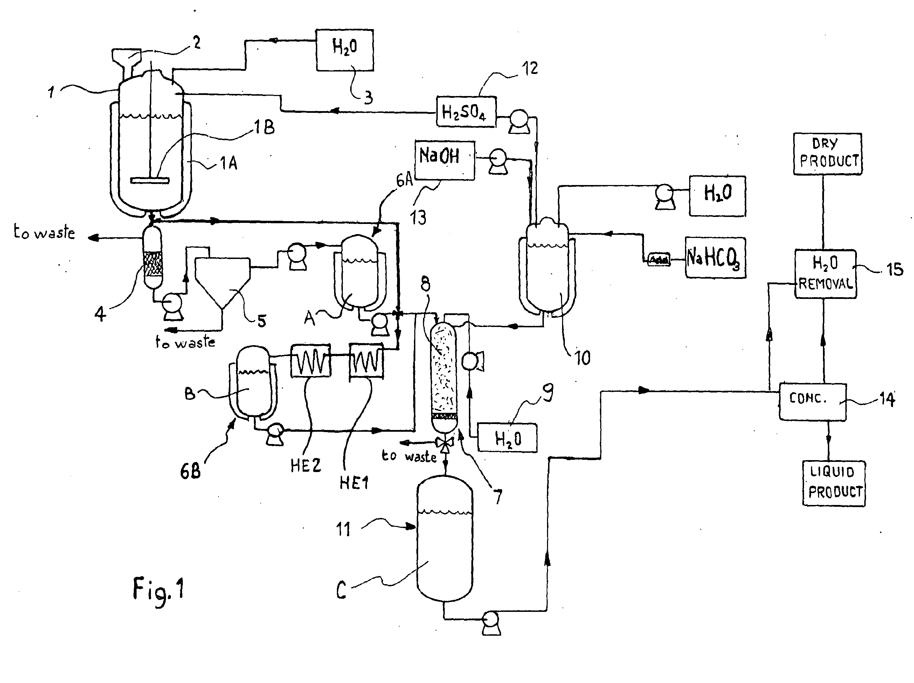

With reference to fig. 1, the invention shows a preferred apparatus, i.e. a plant, for water extraction of pomegranate health promoting phytochemicals, that comprises a blending means, or mill, 1, in which the extraction according to the invention process is carried out.

The mill 1 is provided with feeding means 2 for feeding to it the starting materials, namely the whole pomegranate fruits, i.e. the entire fruit comprising skin, husk, pulp, seeds etc, preferably previously cut in two halves or cut into pieces, for an easier operation of the blades 1B of the mill/blender 1.

As previously mentioned, a treatment with water at a pH within the range of 0.5 to 1.5, for 0.5 to 2.0 hours, preferably about 1 hour, can be carried out on the fruits; the fruits can be treated before being blended in water, during the extraction step, i.e. when stirred and blended in the mill, or after the extraction/blending step.

In the shown embodiment, mill 1 is connected to a supply of water 3; usually, the ratio of water (either neutral and acidic) to solids is within the range of 1:1 to 1:2 v/w, i.e. between 1.0 and 0.5 liters of water per 1.0 Kg of wet pomegranate. As already mentioned, the extraction is carried out on the entire fruit, including seeds and pulp, and the juice of the fruit is therefore mixed in the dispersion, or suspension, of fruit particles obtained by the mill.

If blending is carried out with acidic water, this is generally obtained with a previous acidification of the water by adding an acid, preferably sulphuric acid from container 12, being the final acid concentration within the range 0 to 1 % (w/V water). The pH of acidic water is within the range of 0.5 to 1.5 and the final pH of the blended mixture is 1.0 to 2.0. The blending step is carried out at a temperature within the range of 4 to 30°C, preferably at room temperature within the range of 10 to 25°C; mill 1 is provided with a jacket 1A for temperature control, when necessary.

As already mentioned, the extraction is carried out in water, without any organic solvent, i.e. in the absence of organic solvents.

Suitable blenders, or mills, are e.g. those providing from 1,000 to 12,000 rpm of blades 1 B; the blender motor is cooled to avoid overheating, if necessary. The stirring and disintegrating step of the blender is carried out until a substantially uniform suspension is obtained. In a plant, the stirring time is generally within 2.0 to 2.5 hours for an amount of 60 to 70 kg pomegranate to be treated. The particle size of the solids after blending is within the range of 5 mm to 0.5 mm, and provides an homogeneous suspension of the fruits in acidified water.

Once the acidic extraction is finished, the extract is readjusted to a pH within the range of 3.5 to 5.0 by adding a base, preferably NaOH.

The outlet of mill 1 is connected with a filter 4 for removing solids from the mixture containing ellagitannins and other phenols. The filtered portion is then sent to a further separation means, preferably a centrifuge 5, where further insoluble solids are removed from the reaction mixture to obtain a liquid substantially free from solids and suitable for the following purification and concentration steps. The clarified liquid A, after centrifugation, has a red-brownish colour and is preferably stored in reservoir 6A which is connected to thermal treatment means HE1, HE2.

In the shown preferred embodiment, the clarified liquid A is subjected to a thermal treatment at a temperature not exceeding 100°C, preferably within the range from 70°C to 100°C and most preferably, in the range of 80°C to 90°C by continuous heating of the clarified aqueous solution by using a first heat exchanger HE1, e.g. a tubular heat exchanger, at a residence time within the range of 1 second to 120 second and most preferably in the range from 5 seconds to 30 seconds. The pH of the clarified aqueous solution that undergoes the heat treatment is within the range of 1.0 to 5.0 and most preferably in the range from 3.5 to 5.0. The clarified aqueous solution after the heat treatment is immediately cooled at temperature below 60°C, preferably within the range from 20°C to 60°C and most preferably, in the range of 30°C to 40°C by continuous cooling of the clarified aqueous solution by using a second heat exchanger HE2, e.g. a tubular heat exchanger.

The cooled down liquid B, after thermal treatment, is preferably stored in reservoir 6B which is connected to purification means.

The outlet of mill 1 is also directly connected with thermal treatment means HE1, HE2, so that, according to an embodiment of the invention, pomegranate crude extract can be directly fed to the heat treatment without undergoing to clarification.

Purification means comprises at least one chromatographic column 7 of a resin 8 selected from adsorbent resins, preferably non-ionic.

Suitable adsorption resins are based on non-ionic, hydrophobic, macroreticular cross-linked aromatic polymer. Such resins are typically aromatic polymers, such as styrene and divinylbenzene copolymers, which may be cross-linked. Such resins are known and are generally prepared by polymerization of the appropriate monomers. The adsorption resins used as a chromatographic resin for the purification of ellagitannins of the present invention are not particularly limited so long as they can be eluted using a slightly basic aqueous buffer solution.

Examples of preferred adsorption resins include: Amberlite.RTM. XAD-4, XAD-7, XAD-1180, FPX66, XAD-16 and XAD-1600 (available from Rohm & Haas); XUS-40323.00, XUS-40285.00 and XUS-40283.00 (available from Dow Chemical Co.); and SP-700, SP-825, SP850, Diaion HP 10, HP 20, HP 30, HP 40 and HP 50 (available from Mitsubishi Chemical).

These type of resins are particularly suitable for the invention process in view of their very high adsorption capacity for ellagitannins, including punicalagins and of the fact that the adsorbed punicalagins can be recovered by elution with water and little quantity of sodium bicarbonate, only, without any polar solvent such as methanol or ethanol, as was instead required by the prior art techniques using different resins.

Adsorbed punicalagins, polyphenols and flavonoids are recovered substantially quantitatively.

In the shown preferred embodiment, the process of the invention provides for an one-step purification on chromatographic column.

Liquid A, as obtained from the initial steps a) and b) , i.e. extraction and solid separation, or liquid B obtained from the initial steps a) and b) and c) (being c) thermal treatment) is loaded into column 7, containing the adsorption resin 8 as above detailed. The permeate, containing the sugars, is sent to waste treatment (not shown). Demineralized water from water supply 9 is then fed to column 7 to wash the remaining unbound products.

According to the present invention, in order to recover the adsorbed compounds of interest, an elution basic solution is loaded to the column 7. This water solution is a weakly basic water solution, without organic solvents, having a pH within the range of 7.8 to 8.8, preferably of 8.0 to 8.5. The water solution preferably is a buffer solution. Suitable buffers are Na bicarbonate, Na acetate and ammonium acetate. This elution solution is free from organic solvents, namely it does not contain methanol or ethanol as in the known prior art.

In fact the invention is based on the idea of eluting the adsorbed products by changing the pH of the eluting medium (i.e. water) instead of changing the polarity of the eluting medium by using alcohols. Without giving a full or binding explanation, it is believed that when the aqueous basic buffer solution is fed to the column, the change of the pH of the water generates, in a controlled way, repulsion forces between the -OH groups of the ellagitannins and the resin polymers. This results in a selective elution of only the compounds of interest, still without using organic solvents.

The preferred buffer solution is consisting on 0.5 to 4.0 bed volumes of sodium bicarbonate, prepared into elution tank 10, at a concentration within the range of 500 mM to 5 mM. In the most preferred embodiment the elution buffer consist on 2 bed volumes of 50 mM sodium bicarbonate at room temperature. It is loaded into column 7, promoting the elution, by shifting of the pH, of the punicalagins and other ellagitannins and polyphenols adsorbed onto resin 8.

Eventually, demineralized water, 2 to 3 bed volumes, from water supply 9 is then fed to column 7 to complete the elution displacing the remaining unbound products and the eluted liquid is collected in reservoir 11

The thus obtained liquid product (liquid C) has a purity in punicalagins of at least 55%, and generally of at least 70%, the purity being determined as the % of the peak areas in a chromatogram by HPLC at 360 nm. The recovery of retained punicalagins from the resin is at least 85% and is generally at least 90%.

Fig. 1 also shows a source of sulphuric acid 12 that is connected to column 7 for surface activation of resin 8 and a source of NaOH 13 or other suitable base for regeneration of the same.

Liquid C is preferably freed from the salts and concentrated in concentration means 14, preferably by nanofiltration or reverse osmosis, to a punicalagin content that can reach 10% (w/V).

In a further step of the invention process, the liquid extract obtained by the previously discussed steps is dried in dryer means 15, e.g. freeze-dryer, vacuum rotoevaporator or preferably by spray-dryer, to produce a solid final product. This drying step is preferably carried out on liquid product C, after salt removal and concentration as above disclosed. The drying step can in one embodiment make use of carriers suitable for the final use of the dry product; suitable carriers are e.g. maltodextrines, soluble fiber, oat beta-glucan rich soluble fiber, barley beta-glucan rich soluble fiber, lactose, caseinates etcetera.

Suitable drying techniques are known in the art and comprise spray drying (in a preferred embodiment without the use of carriers), freeze-drying and water evaporation under vacuum. The resulting products will have a punicalagins content of 0.5% to 35% (w/w) if a carrier is used, the punicalagins content can reach up to about 65% (w/w) if no carrier is used. It should be noticed that the invention process provides a purified liquid product C that is sugar-free: this results in the great advantage that liquid C can be concentrated and evaporated to a dry powder without carriers.

The extraction products obtained by the invention process are ellagitannins containing products in liquid or solid form and are characterized by having the following characteristics:
- a punicalagins content (as % w/w) of not less than 5% and preferably of not less than 30% and more preferably of not less than 50%.
- a purity of not less than 55% and preferably of not less than 70% and more preferably of not less than 80%.
- an ellagic acid (free acid) content (as % w/w) of not more than 5% and preferably of not more than 2% and more preferably of not more than 1.5%.
- a total phenol content (as % w/w gallic acid equivalent) of not less than 3% and preferably of not less than 20% and more preferably of not less than 35%.
- a solubility in water (as % w/v) of not less than 3% and preferably of not less than 7% and more preferably of not less than 10%.
- a residual organic solvent content of 0 ppm (i.e. the extract is free from any kind of organic solvent).
- a total sugar content (expressed in ppm) of not more than 500 and preferably of not more than 300 and more preferably of not more than 50.
- a content (as % w/w) in anthocyanins of at least 0.1 % and preferably of not less than 1 %.

Another product not according to the invention are punicalin rich pomegranate extracts prepared by tannase hydrolysis of a punicalagins rich extracts as follows.

Pomegranate extract, liquid or solid (after dissolving in water), rich in punicalagins, was supplemented with a 10 % of crude extract containing tannase. The mixture was gently stirred for one night until complete hydrolysis of punicalagins into punicalin and ellagic acid. Afterwards, the enzyme was inactivated by thermal treatment of the product aqueous solution at 100 °C temperature water bath and 20 seconds residence time, followed with cooling down below 40 °C.

Once the thermal treatment was completed, the product aqueous solution, should be loaded on a column containing an adsorption resin. After permeate is discarded, resin is washed with 2 bed volumes of demineralised water to elute the punicalin. Finally purified aqueous extract containing punicalin, could be dried to get a powder by spray-drier.

A further product not according to the invention are the "spent" pastes obtained after separation of the solids from punicalagin containing solution in step b) of the invention process. As a matter of fact, the pastes, i.e. the part of the extraction mixture containing the solids after the aqueous solution has been separated from the extraction mixture, were found to contain polyphenols in an amount sufficient to impart them an antioxidant activity sufficient to justify the paste use as a pronutrient or as feed product for animals, to provide a balanced diet for ruminants, pigs, poultry, horses, birds, domestic pets and other species, where pomegranate polyphenols are beneficial for improving their antioxidant status.

The invention will now be further disclosed with reference to the following non-limiting examples.

### EXAMPLE 1

### Pomegranate ellagitannins extraction from whole fruit, clarification of the aqueous phase

1000 g of a sample of pomegranate fruits are mixed with 1000 ml of demineralized water. The obtained mixture is blended for a few minutes. After that, the aqueous phase is separated from the solid residue, by filtering on a filter. The solid phase, retained on the filter, is washed with 100 ml of demineralized water, and the water coming from this washing operation is collected with the aqueous phase previously recovered. The aqueous phase, approximately 1700 ml, is then centrifuge refined to eliminate solid particles passed through the filter. After solid elimination, 1495 ml of crude aqueous extract, containing 9.62 g of punicalagin α, β anomers, with a HPLC purity of 68.8%, are obtained:

### EXAMPLE 2

### Tannase inactivation by pH extraction with acidified water.

Three samples of 350 g of a sample of pomegranate fruits showing signals to be attacked by filamentous fungi are mixed each one with 275 of demineralized water previously acidified by adding 1.4 g (plot a), 0.7 (plot b) g or 0 g (plot c) of a 98 % sulphuric acid solution respectively. The obtained mixtures are blended for a few minutes and afterwards they were gentle stirred during 1 hour to complete the extraction of the ellagitannins. After that, the pH of every test were measured being 1.5 , 2.5 and 4.1 respectively. An aliquot of every test were analysed by HPLC to assess the occurrence or not of by-products formed by hydrolysis of punicalagins by tannase enzyme as results of the effectiveness of the inactivation procedure.

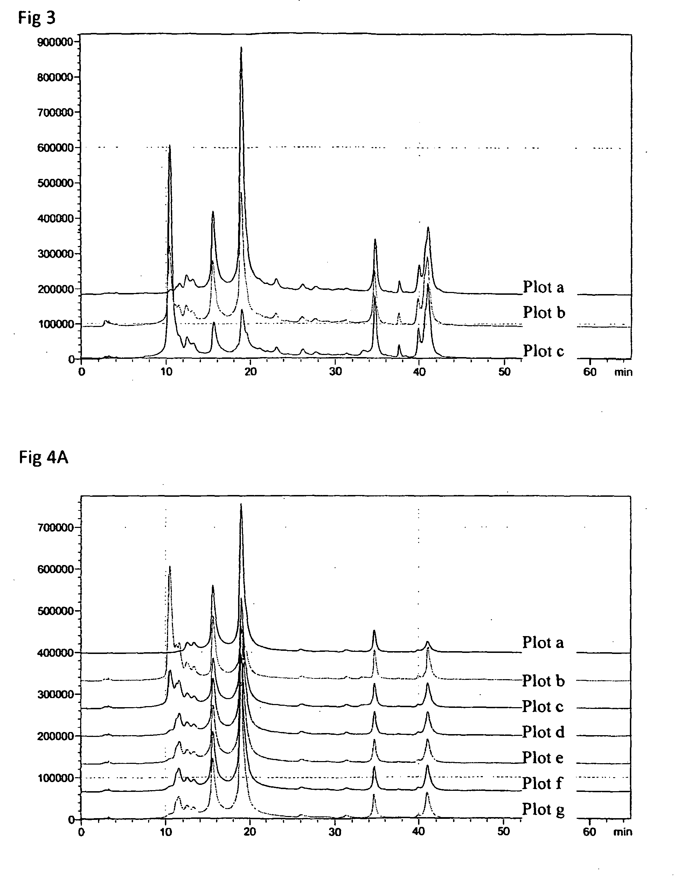

Fig.3 shows the HPLC chromatogram of the analysed samples. In this figure it can be seen that besides the two peaks at 15.5 and 19.0 min, corresponding to the punicalagins, other products related with hydrolysis catalyzed by tannase, punicalin at 10.4 min and free ellagic acid at 41.0 min appearing or increased its concentrations when extraction was carried out at pH 2.5 (plot b) or pH 4.1 (plot c) whereas when extraction was carried out at pH 1.5 (plot a) punicalagins peaks remain intact without signals of hydrolysis and therefore free ellagic acid don't increases its concentration. In conclusion, tannase enzyme could be inactivated by an adequate acidification of the water used for extract the ellagitannins from pomegranate fruits.

### EXAMPLE 3

### Pomegranate ellagitannins extraction from whole fruit with acidified water, clarification of the aqueous phase and spent pomegranate paste preparation.

70 Kg of a sample of pomegranate fruits are mixed with 55 L of demineralized water previously acidified by adding 300 g of a 98 % sulphuric acid solution. The obtained mixture was blended for a few minutes and afterwards it was gently stirred during 1 hour to complete the extraction of the ellagitannins at the same time that the pH inactivation of tannase enzyme was produced. After that, the pH was readjusted to 4.6 by adding 560 g of a 50 %NaOH solution under stirring, and then, the aqueous phase is separated from the solid residue, by filtering on a filter. The solid phase, retained on the filter, is washed with 21.5 L of demineralized water, and the water coming from this washing operation is collected with the aqueous phase previously recovered. The retained solid spent pomegranate paste, 33.3 Kg, is dried until a dry product is obtained, approximately 6.84 Kg, with a moisture of 7.3 %, and analyzed to determine their polyphenolic profile. Table 1 shows concentration in mg/Kg of different plant polyphenols present in the dry spent pomegranate paste.

**Table 1**

| Polyphenols | concentration in mg/Kg |
|---|---|
| Ellagic acid | 3.1 ± 0.3 |
| Punicalagins | 2.9 ± 0.3 |
| Gallic acid | 825.0 ± 91.0 |
| Cafeic acid | 63.6 ± 13.3 |
| Ferulic acid | 23.9 ± 2.8 |
| Coumaric acid | 71.5 ± 7.2 |
| Naringin | 22.4 ± 1.2 |
| Apigenin | 7.0 ± 0.5 |
| Luteolin | 7.9 ± 0.6 |
| Rosmarinic acid | 10.1 ± 1.4 |
| Carnosol | 47.8 ± 7.9 |
| Carnosic acid | 102.5 ± 11.7 |

Additionally the free radical scavenging activity and antioxidant capacity of dry spent pomegranate paste were determined and summarized in Table 2.

**Table 2**

| Assay | Value |
|---|---|
| DPPH assay, lC₅₀ (µM Trolox) | 6.37 |
| ABTS assay (µM Trolox) | 251.8 |
| FRAP assay (mM Fe ²⁺) | 15.4 |

On the other hand, the aqueous phase, approximately 110 L, is then centrifuge refined to eliminate solid particles passed through the filter. After solid elimination, 108 L of crude aqueous extract, containing 408 g of punicalagin α, β anomers, with a HPLC purity of 63.8%, are obtained.

### EXAMPLE 4

### Adsorption punicalagins purification

A sample of 1495 ml of crude aqueous extract containing 9.62 g of punicalagin α, β anomers obtained according to Example 1, is loaded on a column containing a adsorption resin. In this example, XAD 1180 was used but other adsorption resins may be used. The liquid phase recovered at the end of the column, does not contain any punicalagins. After permeate is discarded, resin is washed with 1 bed volume of demineralised water to remove the remaining unbound products. Punicalagins are continuously eluted using a pH shift elution strategy. pH shift is promoted passing through the resin 2 bed volumes of 50 mM sodium bicarbonate. Next, we complete the elution passing demineralised water until at least 90% of the initially loaded punicalagins are recovered. The eluted phase contains approximately 9.42 g of punicalagins with an HPLC purity of about 73.4%.

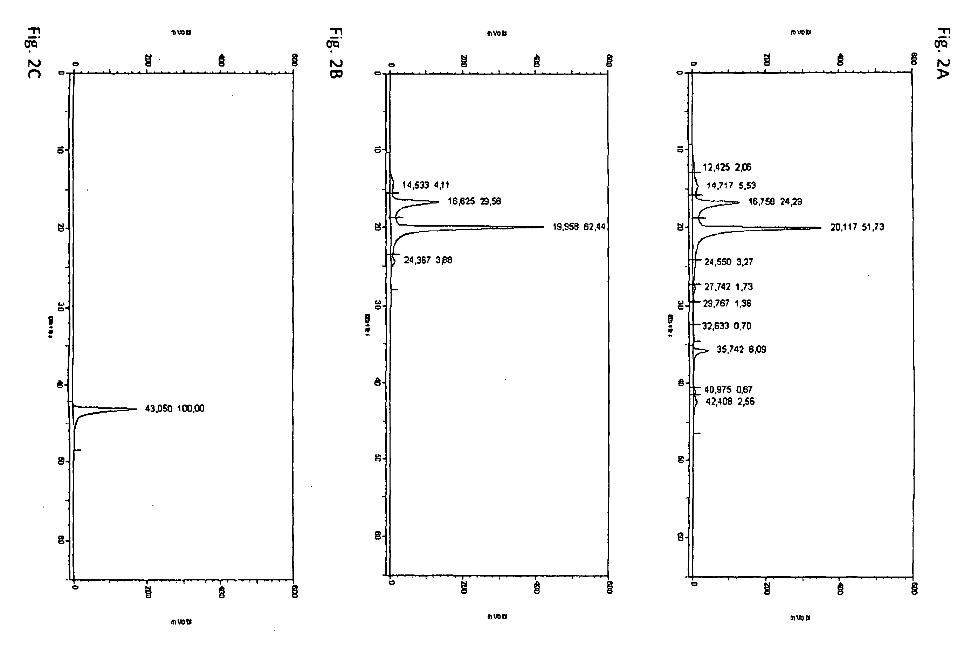

Fig.2A shows the HPLC chromatogram of the obtained purified product. In this figure it can be seen that besides the two peaks at 16 and 20 min, corresponding to the punicalagins, other products are present. Namely, punicalins, ellagic acid glycoside, free ellagic acid, etc.

Figure 2B is the HPLC of punicalagins obtained from the invention process, further purified to provide a standard.

### EXAMPLE 5

### Tannase forming microorganisms and free tannase enzyme inactivation by thermal treatment.

A sample of 350 g of pomegranate fruit showing signals to be attacked by filamentous fungi is mixed with 275 of demineralized water without acidification. The obtained mixture is blended for a few minutes. After that, the aqueous phase is separated from the solid residue, by filtering on a filter. The aqueous phase, is then centrifuge refined to eliminate solid particles passed through the filter.

6 samples of the centrifuge clarified aqueous extract of aprox. 25 mL each were subjected to heat treatment at different heating temperatures. For equilibration of the thermal treatment system, demineralised water was fed at 10.8 mL/min by using a peristaltic pump through a stain steel loop of 600 cm of length and with an internal dead volume of 3.6 mL submerged into a water bath with the desired temperature. Therefore 20 seconds residence time for all the tested temperatures was established as a fix value. Afterwards the hot solution was passed through the 2^{nd} stain steel loop submerged into a water bath at 8 °C to cool down the aqueous extract below 40 °C.

Once the system was equilibrated with water, the first sample was tested at 100 °C for the water bath, and subsequent samples at 90, 80, 70, 60 and 25 °C for the water bath were treated. The initial part, 10 mL, of every sample treatment were discarded and the next 5 mL were collected under aseptic conditions.

A mother solution was then prepared by dissolving in sterile water under aseptic conditions an adequate sample of solid pomegranate extract to reconstitute the extract at a punicalagins content of approximately 4 g/L. Then 25 mL of this mother solution were aliquoted into seven sterile falcon tubes in an aseptic way, and every tube was inoculated with 2.5 mL of:
Plot a: sterile water
Plot b: heat treated sample at 25 °C
Plot c: heat treated sample at 60 °C
Plot d: heat treated sample at 70 °C
Plot e: heat treated sample at 80 °C
Plot f: heat treated sample at 90 °C
Plot g: heat treated sample at 100 °C

Aliquots of every falcon tube were sampled at the day 0 for HPLC analysis and after 2 weeks of incubation into a shaker.

Fig.4A shows the HPLC chromatogram of the analysed samples at day 0. In this figure it can be seen that besides the two peaks at 15.5 and 19.0 min, corresponding to the punicalagins, other products related with hydrolysis catalyzed by tannase, punicalin at 10.4 min and free ellagic acid at 41.0 min appearing or increased its concentrations when heat treatment was carried out at 25 °C (line b, positive control) or at 60 °C (line c) whereas heat treatment was carried out at 70, 80, 90 or 100 °C (lines d, e, f, and g respectively) punicalagins peaks remains intact without signals of hydrolysis and therefore free ellagic acid don't increases its concentration. In conclusion, extracellular tannase enzyme could be inactivated by an adequate thermal treatment.

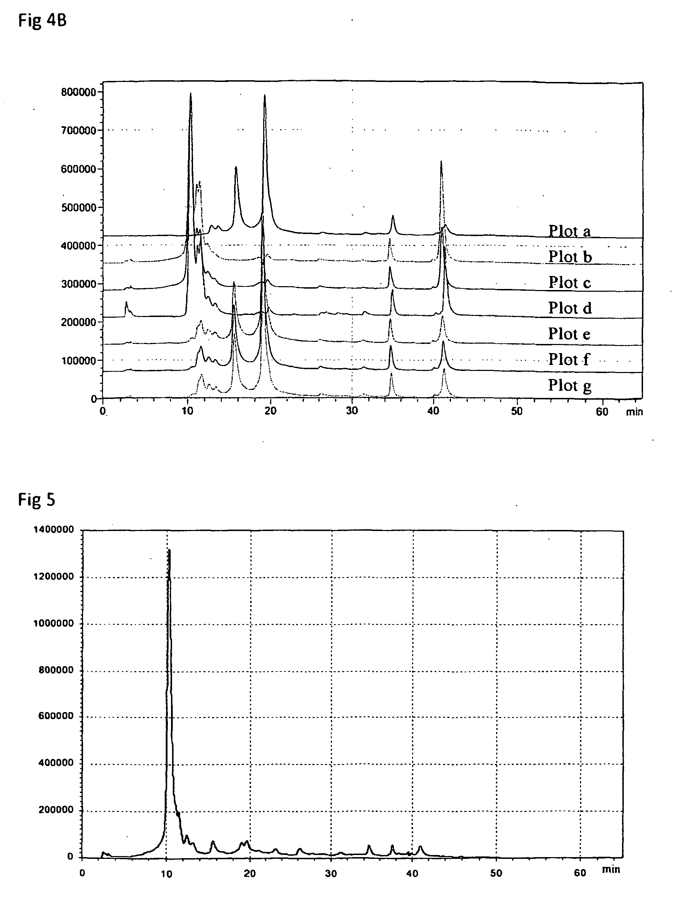

Fig.4B shows the HPLC chromatogram of the analysed samples at day 14. In this figure it can be seen that besides the two peaks at 15.5 and 19.0 min, corresponding to the punicalagins, other products related with hydrolysis catalyzed by tannase, punicalin at 10.4 min and free ellagic acid at 41.0 min appeared or increased its concentration when heat treatment was carried out at 25 °C (line b, positive control), 60 °c (line c) or at 70 °C (line d) whereas when heat treatment was carried out at 80, 90 or 100 °C (lines e, f, and g respectively) punicalagins peaks remain intact without signals of hydrolysis and therefore free ellagic acid does not increases its concentration. In conclusion, tanasse forming microorganisms could be inactivated by an adequate thermical treatment.

### EXAMPLE 6

### Adsorption punicalagins purification after thermal treatment

A sample of 108 L of crude aqueous extract containing 408 g of punicalagin α, β anomers obtained according to Example 3, is fed by using a peristaltic pump through a tubular two heat exchanger system. The 1^{st} tubular heat exchanger increases the temperature of the crude aqueous extract to 90 °C and a 25 seconds residence time at this temperature was established. Afterwards the hot crude aqueous extract was passed through the 2^{nd} tubular heat exchanger to cool down the aqueous extract below 40 °C. The thermally treated aqueous extract was then loaded on a column containing an adsorption resin. In this example, Amberlite FPX66 was used but other adsorption resins may be used. The liquid phase recovered at the end of the column, does not contain any punicalagins. After permeate is discarded, resin is washed with 1 bed volume of demineralised water to remove the remaining unbound products. Punicalagins are continuously eluted using a pH shift elution strategy. pH shift is promoted passing through the resin 2 bed volumes of 50 mM sodium bicarbonate. Next, we complete the elution passing demineralised water until at least 85% of the initially loaded punicalagins are recovered. The eluted phase contains approximately 348 g of punicalagins with an HPLC purity of about 70.5%.

### EXAMPLE 7

### Concentration of pomegranate extract enriched in punicalagins by nanofiltration

A sample of 160 L of thermally treated crude aqueous extract containing 348 g of punicalagins obtained in a pilot plant according to Example 6, is concentrated using a nanofiltration pilot plant equipped with a polymeric membrane, in order to obtain a pomegranate extract concentrate containing 5.8% of punicalagins.

### EXAMPLE 8

### Spray-drying of the purified aqueous extract enriched in punicalagins

A sample of 5.82 I of purified aqueous extract containing 338 g of punicalagins obtained according to Example 7, is slowly fed into a spray-drier. A peristaltic pump is used to feed the spray-dryer, which is previously equilibrated with an inlet air temperature of 135°C. The feeding speed is adjusted in order to obtain an outlet air temperature which is less than 90°C. 595.6 g of an yellowish brown powder, with a moisture of 4.5% (Karl Fischer), an ellagic acid content of 0.4 %, and a punicalagins richness of 56.6%, are obtained.

The following examples refer to a further advantageous aspect of the invention in which pomegranate extract according to the invention is hydrolyzed into punicalin and ellagic acid and punicalin is obtained in high purity and concentration (ex. 9).

Still according to the invention the pomegranate extracts of the invention and/or the punicalin therefrom obtained, are concentrated and dried in the presence of soluble fibers, especially oat and barley beta-glucan rich soluble fibers (ex.10, 11). These soluble fibers act as carriers and are provided in a preferred amount of at least 30%. Preferably the soluble fibers have a beta-glucan (dry basis) content of at least 30 % and within the range of 30% to 60%.

The dry extracts characterized according to claims 9 to 12 are therefore a further object of the invention.

### EXAMPLE 9

### Preparation of a punicalin rich pomegranate extracts by tannase hydrolysis of a punicalagins rich extract

A sample of 100 g of pomegranate extract obtained according to example 8, was dissolved in 1 L of demineralised water. Then 10 mL of a crude extract containing tannase, obtained by previous removal of solids in suspension by centrifugation of a 100 mL sample of solution prepared according example 2, plot c, were added and gentle stirred during one night until practically complete hydrolysis of punicalagins into punicalin and ellagic acid. Afterwards, the enzyme was inactivated by thermical treatment of the product aqueous solution at 100 °C temperature water bath and 20 seconds residence time, followed with cooling down below 40 °C.

Once the thermal treatment was completed, the product aqueous solution, approximately 1000 ml was loaded on a column containing 350 g of XAD 1180 adsorption resin. After permeate is discarded, resin is washed with 2 bed volume of demineralised water to elute the punicalin. The eluted fraction 1 (F1), 700 ml of purified aqueous extract containing punicalin, contains at least 80% of the initially loaded punicalin.

A sample of 350 ml of F1, is slowly feed into a spray-drier. A peristaltic pump is used to feed the spray-dryer, which is previously equilibrated with an inlet air temperature of 150°C. The feeding speed is adjusted in order to obtain an outlet air temperature which is less than 100°C. 29.9 g of a yellowish brown powder, with a moisture of 3.8% (Karl Fischer), a punicalagin content of 0.2 % an ellagic acid content of 0.1 %, and a punicalin content of 47.8%, are obtained.

Fig.5 shows the HPLC chromatogram of the obtained purified product. In this figure it can be seen that peak at 10.4 min, corresponding to punicalin. Ellagic acid formed during the hydrolysis remains bound to the resin because it was not eluted by simply passing water.

### EXAMPLE 10

### Spray-drying of a punicalin rich pomegranate extracts using barley beta-glucan rich soluble fiber as carrier

A sample of 350 ml of a punicalin rich liquid extract (F1) obtained according to Example 9, was added with 33 g of oat beta-glucan rich soluble fiber. Once the fiber was completely dissolved the solution is slowly fed into a spray-drier. A peristaltic pump is used to feed the spray-dryer, which is previously equilibrated with an inlet air temperature of 150°C. The feeding speed is adjusted in order to obtain an outlet air temperature which is less than 100°C. 53.5 g of a yellowish powder, with a moisture of 5.8% (Karl Fischer), and a punicalin richness of 20.8%, are obtained.

### EXAMPLE 11

### Spray-drying of the crude aqueous extract containing punicalagins using oat beta-glucan rich soluble fiber as carrier

A 3 L of crude aqueous extract, containing 11.4 g of of punicalagins obtained according to Example 3, was added with 170 g of oat beta-glucan rich soluble fiber. Once the fiber was completely dissolved the solution is slowly fed into a spray-drier. A peristaltic pump is used to feed the spray-dryer, which is previously equilibrated with an inlet air temperature of 150°C. The feeding speed is adjusted in order to obtain an outlet air temperature which is less than 90°C. 346.1 g of a slightly reddish powder, with a moisture of 5.4% (Karl Fischer), an ellagic content of 0.1 %, and a punicalagins richness of 2.8%, are obtained.

## Claims

1. A process of producing a pomegranate extract comprising the steps of:
a) milling and blending the entire fruit in water to produce a suspension of fruit particles in water;
b) removing solids from the suspension of step a) to obtain a clarified aqueous solution (A);
c) loading the product obtained from step b) in a chromatographic column of an adsorbent non-ionic resin;
**characterized in** further comprising the step of
d) recovering the products retained in said chromatographic column by elution with an aqueous basic solution having a pH within the range of 7.5 to 8.8.

2. A process according to claim 1, wherein said aqueous basic solution is a buffer solution.

3. A process according to claim 1 or 2, further comprising the step of eluting said column with water after elution with said aqueous basic solution

4. A process according to any claim 1 to 3, further comprising the step of treating said fruits in water at a pH within the range of 0.5 to 2.0 before step b) or step c).

5. A process according to claim 4, wherein said blending according to step a) is carried out in water at a pH within the range of 0.5 to 2.0.

6. A process according to claim 4 or 5, further comprising the step of raising the pH of the extraction mixture to a value within the range of 3.5 to 5.0 before carrying out said purification step c).

7. A process according to any previous claim, further comprising the step of:
e) removing water and salts from the liquid product eluted from said resin.

8. A process according to any previous claim further comprising before step b) or step c) the steps of:
- heating the product at a temperature within the range from 70°C to 100°C for a time within the range of 1 second to 120 seconds and subsequently cooling said heated product to a temperature below 60°C, preferably within the range from 20°C to 60°C.

9. A liquid or solid pomegranate extract as obtainable through a process according to any claim 1 to 8, **characterized in** having:
a punicalagins content of at least 30 % (w/w) with a punicalagins purity of at least 70 % (by HPLC 360nm);
a content of ellagic acid of not more than 1.5 % (w/w);
a content of antocyanins of at least 0.1 % (w/w);
a total phenol content (as % w/w gallic acid equivalent) of at least 35%;
a total sugar content of not more than 500 ppm;
a solubility in water (as %w/v) of not less than 7 %.

10. An extract according to claim 9, comprising as carrier a soluble fiber having a beta-glucan (dry basis) content of at least 30 %.

11. An extract according to claim 10, wherein said soluble fiber is selected from one or more of oat beta-glucan rich soluble fiber, barley beta-glucan rich soluble fiber, or their mixture.

12. An extract according to any claim 9 to 11 as depending on claim 8, **characterized in** being free from tannase forming microorganisms and/or free extracellular enzymes able to hydrolize complex ellagitannins.

## Patentansprüche

1. Verfahren zur Herstellung eines Granatapfelextrakts umfassend folgende Schritte:
a) Mahlen und Vermischen der ganzen Frucht in Wasser, um eine Suspension von Fruchtpartikeln in Wasser herzustellen;
b) Entfernen von Feststoffen aus der Suspension von Schritt a), um eine geklärte, wässrige Lösung (A) zu erhalten;
c) Laden des aus Schritt b) erhaltenen Produkts in eine chromatographische Säule eines nichtionischen Adsorberharzes;
**dadurch gekennzeichnet, dass** es des Weiteren folgenden Schritt umfasst:
d) Wiedergewinnung der in der genannten chromatographischen Säule zurückgehaltenen Produkte durch Auswaschen mit einer wässrigen Basislösung, welche einen pH-Wert im Bereich von 7,5 bis 8,8 hat.

2. Verfahren nach Anspruch 1, bei welchem die genannte wässrige Basislösung eine Pufferlösung ist.

3. Verfahren nach Anspruch 1 oder 2, welches des Weiteren den Schritt des Auswaschens der genannten Säule mit Wasser nach dem Auswaschen mit der genannten wässrigen Basislösung umfasst.

4. Verfahren nach einem der Ansprüche 1 bis 3, welches des Weiteren den Schritt des Behandelns der genannten Früchte in Wasser bei einem pH-Wert im Bereich von 0,5 bis 2,0 vor Schritt b) oder Schritt c) umfasst.

5. Verfahren nach Anspruch 4, bei welchem das genannte Vermischen nach Schritt a) in Wasser bei einem pH-Wert im Bereich von 0,5 bis 2,0 durchgeführt wird.

6. Verfahren nach Anspruch 4 oder 5, welches des Weiteren den Schritt des Erhöhens des pH-Werts der Extraktionsmischung auf einen Wert im Bereich von 3,5 bis 5,0 vor dem Ausführen des genannten Reinigungsschritts c) umfasst.

7. Verfahren nach einem der vorherigen Ansprüche, des Weiteren umfassend folgenden Schritt:
e) Entfernen von Wasser und Salzen von dem flüssigen Produkt, welches aus dem genannten Harz ausgewaschen worden ist.

8. Verfahren nach einem der vorherigen Ansprüche, welches des Weiteren vor dem Schritt b) oder c) folgende Schritte umfasst:
- Erhitzen des Produkts auf eine Temperatur im Bereich von 70°C bis 100°C während einer Zeit im Bereich von 1 Sekunde bis 120 Sekunden und anschließendes Abkühlen des genannten erhitzten Produkts auf eine Temperatur unterhalb von 60°C, vorzugsweise im Bereich von 20°C bis 60°C.

9. Flüssiges oder festes Granatapfelextrakt, erhältlich durch ein Verfahren nach einem der Ansprüche 1 bis 8, **dadurch gekennzeichnet, dass** es Folgendes aufweist:
einen Punicalagingehalt von mindestens 30 % (w/w) mit einer Punicalaginreinheit von mindestens 70 % (w/w) (durch HPLC 360 nm);
einen Ellagsäuregehalt von nicht mehr als 1,5 % (w/w);
einen Anthocyangehalt von mindestens 0,1 % (w/w);
einen Gesamtphenolgehalt (als Gallussäureäquivalent in % (w/w)) von mindestens 35 %;
einen Gesamtzuckergehalt von nicht mehr als 500 ppm;
eine Wasserlöslichkeit (% (w/v)) von nicht weniger als 7 %.

10. Extrakt nach Anspruch 9, welches als Träger eine lösliche Faser umfasst, welche einen Beta-Glucangehalt (Trockenbasis) von mindestens 30 % hat.

11. Extrakt nach Anspruch 10, bei welchem die genannte lösliche Faser aus einer oder mehreren einer Hafer-Beta-Glucan-reichen, löslichen Faser, einer Gerste-Beta-Glucan-reichen, löslichen Faser, oder einer Mischung von beiden, ausgewählt ist.

12. Extrakt nach einem der Ansprüche 9 bis 11 in Abhängigkeit von Anspruch 8, **dadurch gekennzeichnet, dass** es frei von tannasebildenden Mikroorganismen und/oder freien extrazellulären Enzymen ist, welche komplexe Ellagitannine hydrolysieren können.

## Revendications

1. Une méthode pour produire un extrait de grenade comportant les étapes suivantes :
a) broyer et mélanger le fruit complet dans de l'eau pour obtenir une suspension de particules de fruit dans l'eau ;
b) enlever les solides de la suspension de l'étape a) pour obtenir une solution aqueuse clarifiée (A) ;
c) verser le produit obtenu de l'étape b) dans une colonne chromatographique d'une résine non ionique absorbante ;
**caractérisée en ce qu'**elle comporte en plus l'étape de
d) récupérer les produits retenus dans cette colonne chromatographique par élution avec une solution basique aqueuse ayant un pH entre 7.5 et 8.8.

2. Une méthode conformément à la revendication 1, dans laquelle cette solution basique est une solution tampon.

3. Une méthode conformément à la revendication 1 ou 2, comportant en plus l'étape d'éluer cette colonne avec de l'eau après l'élution avec cette solution aqueuse basique.

4. Une méthode conformément à une quelconque des revendications 1 à 3, comportant en plus l'étape de traiter ces fruits dans de l'eau à un pH d'entre 0.5 à 2.0 avant l'étape b) ou l'étape c).

5. Une méthode conformément à la revendication 4, dans laquelle ce mélange conformément à l'étape a) se fait dans de l'eau à un pH entre 0.5 et 2.0.

6. Une méthode conformément à la revendication 4 ou 5, comportant en plus l'étape d'élever le pH du mélange d'extraction à une valeur entre 3.5 et 5.0 avant d'effectuer cette étape de purification c).

7. Une méthode conformément à une quelconque des revendications précédentes comportant en plus l'étape de:
e) enlever l'eau et les sels du produit liquide élué de cette résine.

8. Une méthode conformément à une quelconque des revendications précédentes comportant avant l'étape b) ou l'étape c), les étapes de:
- chauffage du produit à la température entre 70°C et 100° C pendant 1 à 120 secondes et ensuite refroidir ce produit chauffé à une température de moins de 60° C, de préférence entre 20°C et 60°C.

9. Un extrait de grenade liquide ou solide pouvant être obtenu à travers la méthode conformément à une quelconque des revendications 1 à 8, **caractérisé en ce qu'**il possède:
une teneur en punicalagins d'au moins 30% (p/p) et une pureté de punicalagins d'au moins 70% (par HPLC 360nm);
une teneur en acide ellagique de pas plus de 1,5% (p/p):
une teneur en anthocyanines d'au moins 0, 1 % (p/p)
une teneur totale en phénols (comme % p/p équivalent à l'acide gallique) d'au moins 35%;
une teneur totale en sucre de pas plus de 500 ppm;
une solubilité dans l'eau (comme % p/p) de pas moins de 7%

10. Un extrait conformément à la revendication 9, comportant comme véhicule une fibre soluble ayant une teneur en béta-glucane (base sèche) d'au moins 30%.

11. Un extrait conformément à la revendication 10, dans lequel cette fibre soluble est sélectionnée d'une ou plusieurs fibres solubles riches en béta-glucane d'avoine, fibre soluble riche en béta-glucane d'orge ou leur mélange.

12. Un extrait conformément à une quelconque des revendications 9 à 11 dépendant de la revendication 8, **caractérisé en ce qu'**il est dépourvu de microorganismes formant le tannasse et/ou dépourvu d'enzymes extracellulaires capables d'hydrolyser des ellagitannins complexes.
